# EUROPÄISCHE PATENTSCHRIFT

(11) **EP 2 343 097 B1**
(45) Veröffentlichungstag und Bekanntmachung des Hinweises auf die Patenterteilung: **20.04.2016**
(21) Anmeldenummer: 11000038.7
(22) Anmeldetag: 05.01.2011
(51) Int. Cl.: A61M 16/00, A61H 31/00

(54) **Vorrichtung zur Generierung eines Atemgashubes während der Herz-Druckmassage**
Device for generating a breathing gas pulse during cardiac massage
Dispositif pour génération d'une pulsation de gas respiratoire lors du massage cardiaque

(30) Priorität: 11.01.2010 DE 102010004403
(43) Veröffentlichungstag der Anmeldung: 13.07.2011
(73) Patentinhaber: Weinmann Emergency Medical Technology GmbH + Co. KG., 22525 Hamburg (DE)
(72) Erfinder: Dietz, Florian, Dr., 23554 Lübeck (DE)
(74) Vertreter: Patentanwälte Klickow & Partner Partnerschaftsgesellschaft mbB

(56) Entgegenhaltungen:
- EP-A1- 2 198 823
- WO-A1-00/20061
- WO-A1-2009/099380
- US-A- 4 326 507
- US-A1- 2006 089 574

## Beschreibung

Die Erfindung betrifft eine Vorrichtung zur Verwendung während der Herz-Druckmassage (HDM) eines Patienten, wobei die Vorrichtung aufweist: eine Atemgasquelle und Mittel, um Atemgas zum Atemweg des Patienten zu leiten (PI), sowie Sensormittel, um eine Thoraxkompression festzustellen wobei sie zudem Steuermittel aufweist, welche einen Atemgashub auslösen, wenn die Sensormittel eine Thoraxkompression registriert haben oder eine solche als in Kürze anstehend registriert haben, wobei die Atemgasquelle den Atemgashub während einer Thoraxkompression im Wesentlichen aufrecht erhält und in der Zeitspanne bis zur nächsten Kompression eine kontrollierte Entlastung der Atemwege durch Vorgabe eines PEEP-Druckes erfolgt.

Aus der WO2009/099380 ist bereits eine Vorrichtung zur Verwendung während der Kardiopulmonalen Wiederbelebung eines Patienten bekannt, welche eine Atemgasquelle mit dem Zyklus der Kardiopulmonalen Wiederbelebung (CPR) synchronisiert. Dazu weist die Vorrichtung eine Atemgasquelle mit einen Tubus im Atemweg des Patienten auf, sowie ein Schaltventil um die Atemgaszufuhr zu regeln und zudem Steuermittel, welche die Aktivierung des Schaltventiles gegenläufig ist mit dem Zyklus der Kardiopulmonalen Wiederbelebung synchronisieren. Es wird vorgeschlagen, den Gasfluss 25% - 2% vor Start CPR zu initialisieren oder 25% - 48% bzw. 52% - 80% nach Beendigung der CPR.

In der EP2198823A1 wird ein Beatmungsgerät zur Verwendung während der Kardiopulmonalen Wiederbelebung (CPR) eines Patienten beschrieben, wobei das Beatmungsgerät sensorisch die Kardiopulmonalen Wiederbelebung qualitativ und quantitativ erfasst und dem Helfer signalisiert, damit dieser die Kardiopulmonale Wiederbelebung besser kontrollieren kann. Zudem bietet das beschriebene Beatmungsgerät die Möglichkeit mit dem Zyklus der CPR synchronisiert zu werden, um nach 15 oder 30 CPR Beatmungshübe auszulösen.

Die WO00/20061 beschreibt eine Methode zur CPR, bei der wiederholt der Brustkorb des Patienten niedergedrückt wird, wobei jede der CPR sensorisch erfasst wird und einem Kontroller übermittelt wird. Nach einer vorbestimmten Anzahl an CPR wird der Patient periodisch beatmet.

Die US 2006/0089574 beschreibt, dass eine Ermittlung der myokardialen Aktivität erfolgt und dass die CPR mit der ermittelten myokardialen Aktivität synchronisiert wird. Es wird auch beschrieben, dass eine Beatmung basierend auf der ermittelten myokardialen Aktivität erfolgen kann. So wird vorgeschlagen, die Inspiration mit der Systole der Herzaktivität zu synchronisieren und die Exspiration mit der Diastole.

Die US 4,326,507 beschreibt eine Vorrichtung zur Verwendung während der Herz-Druckmassage eines Patienten, die kombiniert ist mit einer Vorrichtung zur Ausführung von Zyklen der Herz-Druckmassage. Die Zyklen bestehen aus einer systolischen Phase - bei der der Thorax komprimiert wird - und aus einer Phase der Entlastung des Thorax. Während zumindest eines Zyklus der Herz-Druckmassage wird dabei ein Beatmungsdruck an die Lunge appliziert, wobei ein Ausatemfluss durch ein Ventil verhindert wird, damit der intrathorakale Druck ansteigt. In einem Ausführungsbeispiel erfolgt die Druckzufuhr durch das Beatmungsgerät für drei vollständige Zyklen der Herz-Druckmassage danach wird der Beatmungsdruck für zwei Zyklen der Herz-Druckmassage entlastet.

Die objektive zu lösende Aufgabe ist es somit den nächstliegenden Stand der Technik derart zu modifizieren, dass während eines Zyklus der Herz-Druckmassage die Atemwege ventiliert werden.

Diese Aufgabe wird erfindungsgemäß dadurch gelöst, dass die Atemgasquelle den Atemgashub während einer Thoraxkompression im Wesentlichen aufrecht erhält und in der Zeitspanne bis zur nächsten Kompression eine kontrollierte Entlastung der Atemwege durch Vorgabe eines PEEP-Druckes erfolgt.

Die Vorrichtung weist Sensormittel auf, welche die Signale, welche für eine Thoraxkompression indikativ sind, aufnehmen und einer Auswerteeinheit zuführen. Diese analysiert das Signal auf Schwellwertüberschreitung und löst bei Schwellwertüberschreitung einen Steuerimpuls aus. Dieser wird über eine Steuerleitung, die als Kabel oder auch drahtlos ausgeführt sein kann weitergeleitet. Der Steuerimpuls kann an die Atemgasquelle geleitet werden, welche daraufhin einen definierten Atemgashub an die Luftwege des Patienten abgibt. Der Steuerimpuls kann alternativ an eine Signaleinrichtung geleitet werden, welche den Takt einer durchzuführenden HDM vorgibt. Der Steuerimpuls kann schließlich auch an eine Vorrichtung zur Thoraxkompression geleitet werden, woraufhin diese eine HDM durchführt.

Erfindungsgemäß wird ein neuartiger Beatmungsmodus CCS (Chest Compression Support) vorgeschlagen. Bei dem Beatmungsverfahren für die Reanimation wird zeitlich abgestimmt, bevorzugt synchron, zur manuellen oder maschinellen Thoraxkompression ein Atemgashub ausgelöst. Hier wird das Beatmungsgerät genutzt, um nach Detektion einer Thoraxkompression mittels eines mikrocontroller-gesteuerten Steuermittels die laufende Kompression optimal durch entsprechende Atemgashübe zu unterstützen. Aufgabe des CCS-Modus ist eine schnelle Abgabe von Gas in die Lungen, das nachfolgende Aufrechterhalten eines vorgegebenen Drucks oder eines Gasflusses und ein zeitgerechtes Beenden des Atemgashubes, damit während der Dekompression des Thorax ein möglichst geringer Druck in den Lungen herrscht. Dabei stellt der CCS-Modus sicher, dass überschüssiges Gas oder hoher Beatmungsdruck an die Umgebung abgegeben werden (entweichen) kann.

Es ist auch ein Detektor mit Anzeige für die Bemühungen der Herzdruckmassage des Anwenders vorgesehen. Diese Feedback-Einrichtung liefert optische und/oder akustische und/oder haptische Angaben über die Herzdruckfrequenz und eine möglicherweise sinnvolle Verringerung oder Erhöhung der Kompressionsfrequenz. Zusätzlich ist eine ebenso optische und/oder akustische und/oder haptische Rückmeldung über die Kompressionstiefe vorgesehen.

Ziel der Erfindung ist eine Atemgasquelle für den Einsatz in der Notfallmedizin, mit deren Hilfe eine signifikante Verbesserung des Überlebens nach einer Reanimation mit guten neurologischen Prognosen möglich wird.

Die Atemgasquelle zeichnet sich durch eine neuartige thoraxkompressionssynchrone Beatmung aus:
- Ermittlung des Vorliegens einer Thoraxkompression während der Herzdruckmassage (HDM) und
- Abgabe eines Atemgashubes synchron oder zeitlich versetzt zur Thoraxkompression

Dadurch wird die Pumpleistung der Thoraxkompression durch zeitgleiches bzw. zeitnahes pneumatisches Gegendrücken in der Lunge durch den Atemgashub wesentlich erhöht und der Organismus wird verstärkt mit Blut und Sauerstoff versorgt.

Darüberhinaus ist eine Unterstützung des Anwenders während der manuellen Herzdruckmassage durch ein Rückmeldesystem, welches Anweisungen zur Anwendungsfrequenz und -stärke angibt, vorgesehen.

Es ergibt sich eine Unterstützung und Verbesserung des gesamten Prozesses der Reanimation dadurch, dass das Rettungspersonal von der Beatmungsaufgabe mittels Beatmungsbeutel entbunden ist, wodurch eine verbesserte Wirkung der externen Herzdruckmassage erreicht wird.

Die Atemgasquelle kann innerhalb von wenigen Millisekunden einen relevanten Atemgasdruck von bis zu 60mbar oder einem anderen relevanten Atemwegsdruck zwischen 10 und 80mbar aufbauen und weiterhin innerhalb einer Kompressionsphase von ca. 300ms einen vorgegebenen Druck oder Gasfluss aufrecht erhalten. Schließlich ermöglicht das Gerät die Abgabe von 100% Sauerstoff für die Insuflation der Lungen.

Zur Erfindung gehört auch ein Sensormittel für Thoraxkompressionen, welches es ermöglicht manuell oder maschinell ausgeführte Thoraxkompressionen innerhalb von wenigen Millisekunden zu erkennen. Dieses Sensormittel ermöglicht gemeinsam mit der Atemgasquelle eine schnellstmögliche Reaktion auf die Thoraxkompression im Sinne der Abgabe eines Atemgashubes. Der Gasstrom wird dem Patienten entweder mit konstantem Druck zugeführt oder beginnend mit einem höheren Druck, welcher dann auf ein niedrigeres Druckniveau abgesenkt wird.

Das Absenken und die Erhöhung des Beatmungsdruckes werden dabei an Hand verschiedener, durch das Gerät und über messtechnisch erfasste Atemparameter identifizierter, Ereignisse getätigt.

Eine Synchronisation zwischen Herzdruckmassage und Beatmung erfolgt über intelligente und robuste Triggeralgorithmen, wodurch Fehltrigger oder Autotrigger vermieden werden können.

Eine Schädigung des Patienten durch zu hohe Drücke oder Volumina wird durch eine patienteninterface-abhängige Begrenzung des Beatmungsdruckes und/oder eine alternative Auswahl von druck- oder volumenkontrollierter Beatmung erreicht.

Eine individuelle Anpassung des Atemgashubes in Relation zur Thoraxkompression wird dadurch erreicht, dass messtechnisch das Ausmaß der Thoraxkompression bestimmt wird und ein variabler Atemgashub entsprechend dem Ausmaß der Kompression ausgelöst wird.

Als einfaches und robustes Triggersignal für das Auslösen des Atemgashubes erfolgt die Nutzung von respiratorischen Messgrößen wie Atemgasdruck und/oder Atemgasfluss und/oder Atemgasvolumen und/oder exspiratorisches CO2.

Die Höhe des Druckes bzw. des Volumenstromes während des maschinellen Beatmungshubes wird dadurch ängepasst, dass eine druck- oder volumenkontrollierte Beatmung vorgesehen ist, welche unterschiedliche Auswirkungen auf das Tidalvolumen haben.

Die Empfindlichkeit und Ausprägung des Triggers zum Beginn eines maschinellen Atemgashubes, also der Zeitversatz zwischen Kompression und Atemgashub, sind einstellbar.

Die Empfindlichkeit und Ausprägung eines Triggers zur Beendigung des maschinellen Atemgashubes ist ebenso einstellbar.

Als Atemgasquelle kommen verschiedene derzeit bekannte Gerätetechnologien zum Einsatz: Beatmungsgeräte mit Gebläse oder Kompressor und/oder druckgasgespeiste Beatmungsgeräte und/oder Servoventile und/oder Schaltventile und/oder Blenden.

Als wichtige Rückführgrößen stehen die Anwendungsfrequenz und Herzdrucktiefe/-stärke sowie die vollständige Entlastung während der Dekompression zur Verfügung.

Die Erfindung besteht aus einer Atemgasquelle, welche einen Atemgashub, also Atemgas mit definierbarem Druck und/oder Fluss und/oder Volumen über ein Ventil oder einen anderen Aktor (z.B. hochdynamisches Gebläse) zu einem Patienteninterface (PI) liefert. Die Atemgasquelle kann den Atemgashub über einen definierten Zeitraum aufrecht erhalten, beispielsweise für die Zeitdauer einer Thoraxkompression. Ein weiterer Atemgashub wird erst ausgelöst nachdem die Sensormittel (23a, 23b) die Beendigung einer Thoraxkompression registriert haben.

Das Ventil bzw. der Aktor befindet sich im Bereich zwischen einer Druckgasquelle oder Umgebungsluft und dem Patienten und dient dazu den Atemgashub zu steuern bzw. auch zu unterbrechen.

Ein Sensormittel zur Erfassung eines Atemgasparameters beispielsweise Druck und/oder Flow und/oder Volumen und/oder Temperatur und/oder Feuchtigkeit und/oder 02 und/oder CO2 und/oder Beschleunigung ist patientennah im Patienteninterface oder im Bereich der Atemgasquelle vorgesehen.

Zudem ist ein Sensormittel im Bereich des Applikationsortes der Thoraxkompression (Sternum) angeordnet, um dort die Kompression des Thorax oder Signale, die indikativ für die Thoraxkompression sind, zu registrieren. Als Signale oder Messprinzipien sind alternativ oder ergänzend vorgesehen: Beschleunigung und/oder Kraft und/oder Druck und/oder barometrischer Druck und/oder Triangulation und/oder direkte Wegmessung über Winkelsensoren oder Ultraschall und/oder als mechanischer Schalter und/oder durch Messung der Körperimpedanz durch Defi- oder EKG- oder RIP-Elektroden und/oder NIBP und/oder invasive Blutdruckmessung und/oder über invasiv oder nicht-invasiv gemessene cranielle, arterielle, venöse, gemischt-venöse oder zentralvenöse Sauerstoffsättigungen und/oder über die Pulswelle.

Die Sensorsignale werden über eine Recheneinheit (Steuermittel) elektronisch oder pneumatisch ausgewertet und zur Ansteuerung des Ventils oder der Atemgasquelle genutzt, um in Antwort auf Sensorsignale einen Atemgasimpuls (Druck, Volumen, Fluss) in Richtung des PI abzugeben.

Als Patienteninterface (PI) sind vorsehbar: Endotrachealtubus oder Tracheostoma oder Maske oder Kombi-Tubus oder Larynxmaske oder nasal pillow. Bei Verwendung von Endotrachealtubus oder Tracheostoma oder Kombi-Tubus sind Drücke bis 60 mbar sinnvoll. Bei Verwendung anderer PI, wegen der Gefahr einer Magenüberblähung Drücke bis max. 20 mbar.

Im Bereich des PI kann ein Exspirations-Ventil - zur Aufrechterhaltung eines PEEP - oder ein Ausatemsystem oder ein Mehrschlauchsystem vorgesehen sein.

Bei Gebläsebeatmung ist wahlweise zu einer hochdynamischen Ausführung des Gebläses ein Ventil zwischen Gasquelle und Patient vorgesehen. Weiterhin ist wahlweise ein Ventil zwischen Gasquelle oder Umgebungsluft und Gebläse vorgesehen.

Typisch wird das Atemgas über einen Schlauch zum PI geführt. Alternativ kann die Gasquelle auch unmittelbar am PI vorgesehen sein.

Das entsprechende Verfahren sieht vor, dass Beginn und Ende der Thoraxkompression messtechnisch ermittelt werden. Der Beginn wird beispielsweise durch Schwellwertüberschreitung eines oder mehrerer Sensorsignale (ggf. mit Verrechnung der Sensorsignale) ermittelt. Im einfachsten Fall drückt der Anwender einen Schalter nieder, wenn er eine Thoraxkompression durchführt.

Die Sensorsignale werden unmittelbar zur Signalextraktion verwendet oder auch in der 1. und/oder 2. und/oder 3. Ableitung oder Integration von Sensorsignalen.

Der Zeitverlauf von Signalen wird mittels Filtern oder adaptiven Verfahren oder wissensbasierten Methoden ermittelt.

Ist der Beginn der Kompression erfasst, so wird ein Steuersignal für die Atemgasquelle und/oder das Ventil generiert, um einen Atemgashub freizugeben.

Der Beginn der Abgabe von Atemgas kann zu Beginn der Thoraxkompression erfolgen oder auch zeitversetzt beispielsweise 10, 20, 30 ... 90, 100 ....130, 140, 150, ..., 430, 440, 450,...,500msec nach dem Beginn der Kompression.

In einer Ausführungsform ist auch daran gedacht, dass der Beginn der Abgabe von Atemgas zeitgesteuert erfolgt beispielsweise alle 600 msec, um eine Präoxygenierung zu erreichen und für den Anwender ein Signal für die Thoraxkompression vorzugeben, in Form eines sich hebenden Thorax (optisches Signal) und in Form einer Gegenkraft bei aufliegenden Händen (haptisches Signal).

Alternativ kann der Beginn der Abgabe von Atemgas auch gerätegesteuert über eine Vorrichtung zur Thoraxkompression erfolgen. Die Vorrichtung zur Thoraxkompression leitet hierbei ein, optional zeitversetzt gesandtes, Steuersignal an die Vorrichtung zur Beatmung, welche aufgrund des Steuersignales einen Atemgashub freisetzt. Das Steuersignal kann auch vor Einsetzen der Thoraxkompression abgegeben werden.

Das Ende des Atemgashubes wird zeitgesteuert z.B. nach 50 ...400 msec eingeleitet oder triggergesteuert nach Schwellwertüberschreitung eines oder mehrerer Sensorsignale (ggf. mit Verrechnung der Sensorsignale) oder wissensbasiert bzw. mit Filtern oder adaptiven Verfahren.

Das Ende des Atemgashubes kann auch gerätegesteuert über eine Vorrichtung zur Thoraxkompression vorgegeben werden.

Die Applikation des Atemgases kann druck- und/oder fluss- und/oder volumengesteuert erfolgen, wobei eine konstante oder variable (abfallend, sinusförmig, trapezförmig ...) Abgabe vorgesehen ist.

Im Ergebnis sollen, wenn eine kardiopulmonale Reanimation durchgeführt wird, das Ausmaß und die Dauer des Überdrucks im Brustraum des Patienten erhöht sein, um den Blutstrom aus dem arteriellen Gefäßsystem und dem Herz zu verstärken.

Eine aktive oder passive oder kontrollierte Entlastung der Atemwege beendet den Atemgashub. Kontrolliert durch eine PEEP Vorgabe oder aktiv über einen negativen Druck.

Der Thoraxdruck verschiebt ein Volumen an Atemgas aus der Lunge zum Gerät. Das Volumen wird sensorisch erfasst zur Messung des Volumens. Das verschobene Volumen ist ein Indikator für die Druckstärke der Herz-Druckmassage (HDM) und somit geeignet als ein Qualitätsindex für die Güte der Thoraxkompression. Über einen Abgleich mit einem initialen Atemgashub ohne Thoraxkompression, welcher dazu dient, die Lungen- und Thoraxeigenschaft zu bestimmen, kann so ein Signal gewonnen werden, welches patientenindividuell die Güte der Thoraxkompression zu ermitteln hilft.

Mit dem initialen Hub lassen sich auch Patientengruppen unterscheiden (Kind, Erwachsen). Daraufhin kann variabel der Atemgashub angepasst werden und ein Feedback Signal für den Anwender erzeugt werden.

Aus dem Feedback können die Frequenz und ein Kompressions- Dekompressions-Verhältnis ermittelt werden und an den Anwender zurückgemeldet werden; z.B. akustisch und/oder optisch und/oder taktil/haptisch. Ebenso kann ermittelt werden, ob der Anwender den Thorax zur Dekompression optimal entlastet.

Erfindungsgemäß ist auch eine Begrenzung der Applikationsenergie des Atemgashubes vorgesehen, im Sinne einer Schutzfunktion für den Patienten. So können Grenzen für Druck und/oder Volumen und/oder Fluss und/oder Dauer vorgegeben werden.

Es ist auch daran gedacht die Gaszusammensetzung zu beeinflussen, so sind Anteile von 100 %, 90% ... 40 % ...21 % 02 möglich. Bevorzugt erfolgt eine Anpassung des 02-Gehaltes über sensorische Messung des SpO2 oder alternative invasive oder nicht-invasive Verfahren zur Ermittlung der Sauerstoffsättigung an verschiedenen Messorten (venös, gemischt-venös, zentral-venös, intra-craniel) oder des arteriellen Sauerstoffpartialdrucks des Blutes.

Bei einer Kompression mit 100 x pro Min wird bei Verwendung des Verfahrens beispielsweise jeweils ein Atemgasvolumen von 100 ml abgeatmet, was ein Minutenvolumen von 10 L/min ergibt. Dadurch wird CO2 massiv abgeatmet in Richtung 20 - 25 mmHg. Es ist daher auch vorgesehen, das Totraumvolumen zu vergrößern, um der Gefahr einer zu großen CO2-Auswaschung (Hypokapnie) entgegenzuwirken. Dazu wird z.B. das Ausatemventil bzw. Ausatemsystem weiter entfernt vom PI platziert oder es wird ein PI mit großem Totraum vorgesehen.

Ein variables Totraumvolumen in Abhängigkeit von der Änderung des etCO2 hilft dem Anwender, das Verfahren auf die aktuelle Situation anzupassen. Dazu ist es erfindungsgemäß vorgesehen, z.B. parallel angeordnete Filter durch Ventile als zusätzliches Totraumvolumen zu- oder abzuschalten.

Eine Thoraxkompression kann auf unterschiedliche Weise erfolgen. Entweder durch einen Helfer, der eine Herzdruckmassage (HDM) durchführt, oder durch eine Vorrichtung zur Thoraxkompression (30) oder auch durch einen Atemgashub, welcher die Lunge des Patienten aufbläht und so den Thoraxraum sozusagen von innen heraus komprimiert. Ziel einer Thoraxkompression ist es immer einen Druckimpuls auf das Herz auszuüben, um den Aufwurf von Blut zu unterstützen.

Ein wichtiger Aspekt der Erfindung ist, dass während der Kompression der Brust des Patienten praktisch kein Atemgas aus der Lunge des Patienten herausströmt, um einen erhöhten Druck im Brustraum während der Kompressionsphase zur Unterstützung des kardiopulmonalen Kreislaufes zumindest aufrecht zu erhalten. Dazu liefert die Atemgasquelle während der Kompressionsphase laufend Atemgas nach oder sorgt mittels Ventilen dafür, dass kein Rückstrom erfolgt.

Die Atemgaszufuhr soll erfindungsgemäß während der Kompressionsphase erfolgen, um den Überdruck im Brustkorb weiter zu erhöhen.

Es ist auch daran gedacht, dass zuerst eine Atemgaszufuhr in die Lungen des Patienten erfolgt, um im Thorax einen erhöhten Innendruck zu erzeugen. Die Atemgasquelle hält den erhöhten Druck dann für zumindest 50 msec oder bevorzugt für die Dauer einer HDM und signalisiert dabei optisch, akustisch oder taktil, dass eine Herzdruckmassage durchzuführen ist. Bevorzugt gibt die Signaleinrichtung auch den Takt für die HDM vor. Somit wäre zeitgleich ein erhöhter Druck in der Lunge und über die HDM von außen auf den Thorax gegeben. Wobei der gleichzeitige Druck von innen über die Lunge und von außen über die HDM, dient dazu das Herz und periphere Gefäße zu komprimieren, um einen Auswurf von Blut zu unterstützen.

In den Zeichnungen sind Ausführungsbeispiele der Erfindung dargestellt. Es zeigen:
- Fig. 1: einen grundsätzlichen Aufbau einer Vorrichtung zur Beatmung,
- Fig. 2: eine schematische Darstellung einer Beatmungseinrichtung mit einer Sensoreinrichtung zur Erfassung von Thoraxkompressionen,
- Fig. 3: einen zeitlichen Verlauf der Eindrücktiefe (E) einer Thoraxkompression,
- Fig. 4: einen Druckverlauf (D) im Thorax gemäß Fig. 3,
- Fig. 5: einen Volumenverlauf (V) in der Lunge unter regelmäßiger Thoraxkompression
- Fig. 6: einen grundsätzlichen Aufbau der Vorrichtung zur Beatmung mit Sensoreinrichtung
- Fig. 7: einen alternativen Aufbau gemäß Fig. 6

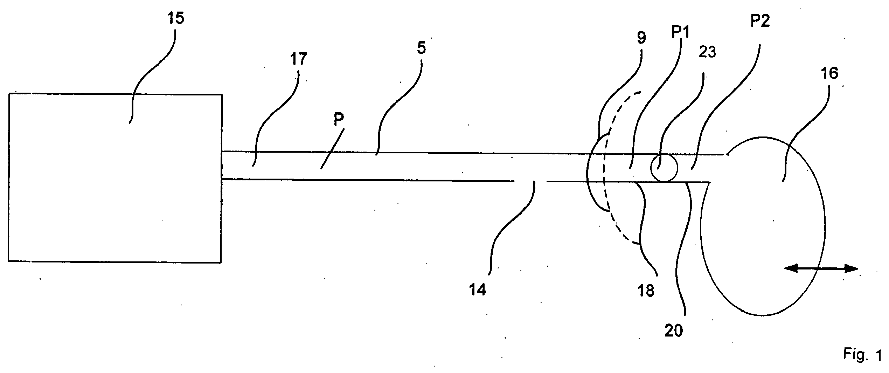

Fig. 1 zeigt den grundsätzlichen Aufbau einer Vorrichtung zur Beatmung (15) Im Bereich eines Gerätegehäuses (1) mit Bedienfeld (2) sowie Anzeige (3) ist in einem Geräteinnenraum eine Atemgasquelle (15) angeordnet. Über ein Kopplungselement (4) wird ein Verbindungsschlauch (5) angeschlossen. Über ein Bedienelement (13) kann das Kopplungselement (4) einfach und schnell an das Beatmungsgerät angekoppelt werden. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf. Im Bereich einer dem Gerätegehäuse (1) abgewandten Ausdehnung des Verbindungsschlauches (5) ist ein Ausatmungselement (14) angeordnet. Über den Beatmungsschlauch (5) kann ebenfalls eine Maske (9) angeschlossen werden, welche durch eine Kopfbänderung (10) am Patientenkopf fixiert wird.

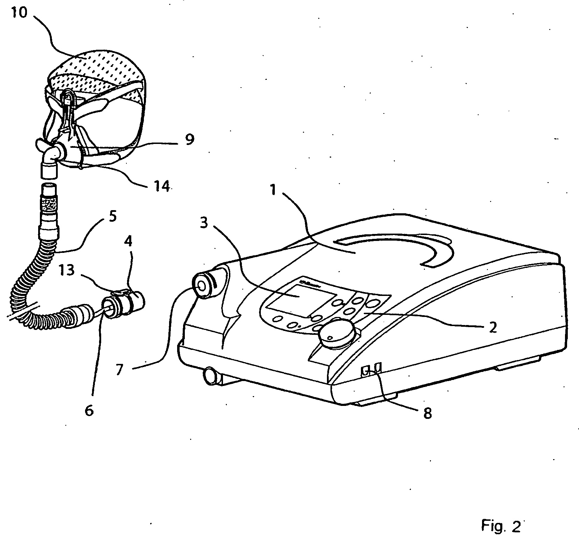

Gemäß der Ausführungsform in Fig. 2 ist ein Beatmungsgerät (15) zur Durchführung von Beatmungen über einen Verbindungsschlauch (5) mit einer Maske (9) verbunden. Der Verbindungsschlauch (5) weist als Ausatemelement (14) eine konstante Öffnung auf. Dargestellt ist ebenfalls eine Lunge (16) eines Patienten. Im Bereich eines Leitungsabschnittes (17) liegt ein vom Beatmungsgerät (15) bereitgestellter Versorgungsdruck P vor. In der Nähe der Maske (9) kann patientenseitig an einem Messort (18) durch einen Sensor (23) ein Druck P1 erfasst werden, der einem PEEP-Druck entspricht.
Die während des Auftretens einer Thoraxkompression hervorgerufenen Exkursionen führen zu Druckänderungen im Thorax und in der Lunge. Während einer Thoraxkompression kann somit der Druck in der Lunge P2 höher sein, als der Druck P1.

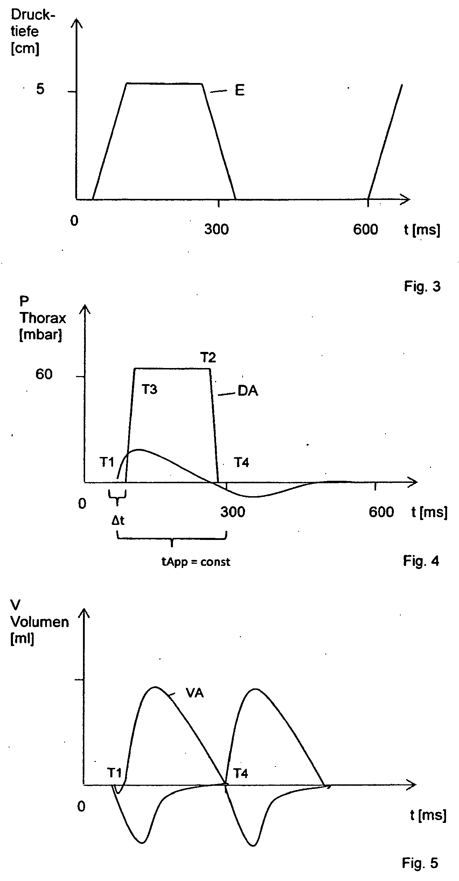

Fig. 3 zeigt den zeitlichen Verlauf der Eindrücktiefe (E) einer Thoraxkompression, der typisch ist sowohl für automatische mechanische Thoraxkompression, als auch für manuelle Bemühungen eines Helfers. Eine typische Thoraxkompression, im Sinne effektiver Reanimationsbemühungen, weist eine Drucktiefe von mindestens 5 cm und eine Zeitdauer von bis zu maximal 300 msec auf. Charakteristisch ist ein rascher Anstieg zur maximalen Drucktiefe.

Fig. 4 zeigt einen Druckverlauf im Thorax (P 2) gemäß der in Fig. 3 vorgenommenen Thoraxkompression. Beginnend mit dem Niederdrücken des Thorax steigt der Druck im Thorax und in der Lunge bis zu einem Maximum an und fällt dann zum Ende der Kompression auf den Ausgangswert zurück. Der Druckverlauf (P2) kehrt sich nach Ende der Kompression um. Es kommt zu einer Entlastung des Thorax, welche bis zum Einsetzen der nachfolgenden Kompression andauert.

Der erfindungsgemäße Sensor der Vorrichtung registriert den Druckanstieg zum Beginn der Kompression. Bei Überschreitung eines Schwellwertes (Trigger) wird ein Steuersignal für die Atemgasquelle (15) generiert. Die Atemgasquelle gibt daraufhin einen Druckhub (DA) an Atemgas frei. Dieser kann im Bereich 10 mbar bis 80 mbar liegen. Die Überschreitung des Schwellwertes erfolgt nach einem Zeitraum Δt. Zum Zeitpunkt T1 wird von der Atemgasquelle der Druckhub freigegeben. Der Druckhub wird in diesem Beispiel konstant für den Zeitraum der Kompression appliziert T3. Das Ende der Kompression wird zum Zeitpunkt T2 sensorisch erfasst. Aufgrund einer Schwellwertunterschreitung wird ein Steuersignal an die Atemgasquelle geleitet, welches den Druckhub beendet. In der Zeitspanne bis zur nächsten Kompression T4 erfolgt eine kontrollierte Entlastung der Atemwege durch Vorgabe eines PEEP-Druckes.

Durch die Thoraxkompression wird in der Lunge ein Atemgasvolumen bewegt und aus der Lunge verschoben. Fig. 5 zeigt einen Volumenverlauf (V) in der Lunge unter regelmäßiger Thoraxkompression. Es liegt zum einen, ebenfalls wie beim Druckverlauf gemäß Fig.4, eine zur Kompression korrespondierende Volumenbewegung vor, die jedoch vorzeichenverkehrt zum Druck erfolgt. Mit Einsetzen der Kompression wird schnell ein Volumen aus der Lunge verschoben. Vor Beendigung der Kompression ist der Volumenstrom auf Null abgefallen. Bei Entlastung kehrt sich der Volumenstrom um, es strömt Atemgas in die Lunge ein.

Die Sensorik registriert den Volumenanstieg zum Beginn der Kompression. Bei Überschreitung eines Schwellwertes (Trigger) wird ein Steuersignal für die Atemgasquelle (15) generiert. Die Atemgasquelle gibt daraufhin einen Volumenhub (VA) an Atemgas frei. Auch hier setzt erst nach einem Zeitraum Δt der Volumenhub (VA) ein. Zum Zeitpunkt T1 wird von der Atemgasquelle der Volumenhub freigegeben, welcher in diesem Beispiel ein vorgegebenes Volumen beinhaltet.

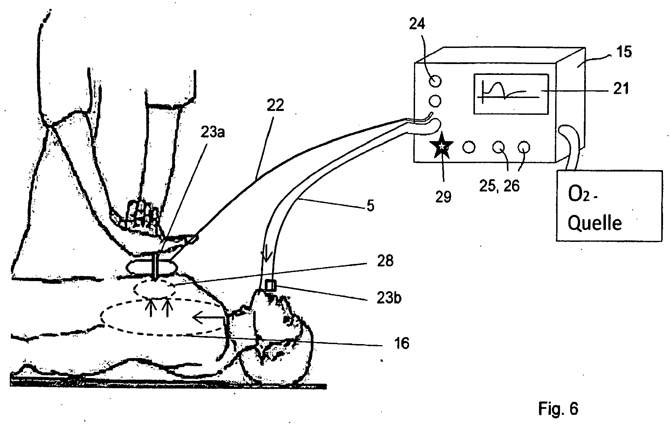

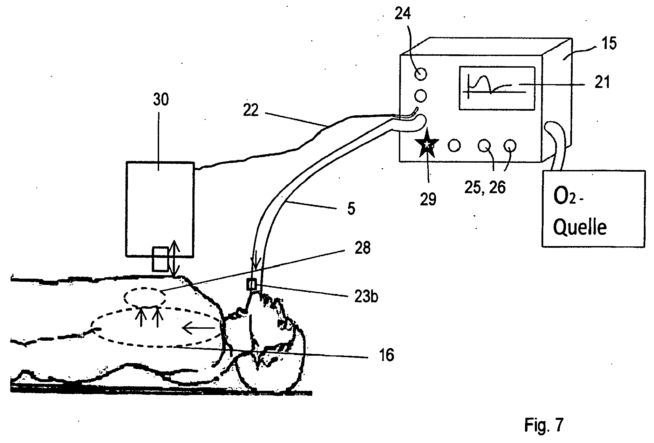

Fig. 6 zeigt einen grundsätzlichen Aufbau der Vorrichtung zur Beatmung (15) mit Sensoreinrichtung (23a, b). Die vom Anwender durchgeführte Kompression (Pfeil) wird von der Sensorik (23a) registriert. Die Sensorik (23a) generiert ein Steuersignal welches über die Signalleitung (22) an das Beatmungsgerät (15) gesendet wird, damit dieses einen Atemgasstoß in Richtung auf den Patienten abgibt (die nächste Inspiration). Die Sensoreinrichtung ermittelt auch die Frequenz und/oder die auf den Brustkorb ausgeübte Kraft und/oder die Eindrücktiefe der HDM und leitet diese Signale an die Signaleinrichtung (29) zur Wiedergabe weiter. Der Betrieb des Beatmungsgerätes (15) ist sowohl mit manueller Herzdruckmassage, über die Sensorik (23a), wie auch mittels einer gekoppelten Vorrichtung zur Thoraxkompression (30) (in Fig. 7 dargestellt) möglich. Diese drückt rhythmisch einen Stempel auf den Thorax des Patienten. Im Falle der Verwendung einer Vorrichtung zur Thoraxkompression (30), dient diese als Sensorik, welche das Steuersignal für das Beatmungsgerät (15) bei Niederdrücken des Thorax generiert und über eine Signalleitung (22) als Steuersignal an das Beatmungsgerät leitet.

Der Vorteil dieser Anwendungsform gegenüber der ersten ist die optimierte Ansteuerung des Beatmungsgerätes ohne mechanische Verzögerungen, welche bei der Übertragung der Kompressionskraft auf Sternum und in der Folge auf die Druckverhältnisse in der Lunge (16), in den Atemwegen und an der Sensorik (23a) zur Auslösung des Triggers entsteht.

Gemäß Fig. 7 erfolgt über die Atemgasquelle (15) zuerst eine Atemgaszufuhr in die Lungen (16) des Patienten, um im Thorax einen erhöhten Innendruck zu erzeugen. Die Atemgasquelle (15) hält den erhöhten Druck dann für zumindest 50 msec oder bevorzugt für die Dauer einer HDM. Dabei wird über die Signaleinrichtung (29) (optisch, akustisch oder taktil) angezeigt, dass eine Herzdruckmassage durchzuführen ist. Bevorzugt gibt die Signaleinrichtung (29) auch den Takt für die HDM vor. Im Takt der Signaleinrichtung drückt der Helfer auf den Thorax. Alternativ generiert die Atemgasquelle (15) bei Atemgaszufuhr über die Signalleitung (22) ein Steuersignal an eine Vorrichtung zur Thoraxkompression (30). Diese führt dann die Thoraxkompressionen solange aus, bis ein Steuersignal der Atemgasquelle (15) die Thoraxkompression beendet. Während der Thorax niedergedrückt ist, ergibt sich, im Thoraxraum, ein erhöhter Druck auf das Herz (28) und das umliegende Gewebe, da zeitgleich ein erhöhter Druck in der Lunge (16) und über die HDM von außen auf den Thorax ausgeübt wird. Der gleichzeitige Druck von innen über die Lunge und von außen über die HDM dient dazu, das Herz und periphere Gefäße zu komprimieren, um einen Auswurf von Blut zu unterstützen.

In einer alternativen Ausführung der Vorrichtung nach Fig. 7 wird ein druckgasbetriebenes Beatmungsgerät (15), welches mittels einer Injektoreinrichtung Gaskonzentrationen von 40-100% 02 ermöglicht, mittels eines Einschlauchsystems mit patientennahem Exspirationsventil an einen Patienten konnektiert. Das Beatmungsgerät kann mit Hilfe seiner patientennah angebrachten Flow-, Volumen-, CO2- und Drucksensorik (23b) den Beginn einer Thoraxkompression messen, indem relative Druckerhöhungen, positive Drucksteigungen, exspiratorische Flüsse · und exspiratorische Volumina und CO2-Konzentrationen erfasst werden. Die Sensorik (23b) ist hier beispielsweise im Bereich des Patienteninterface angeordnet und kabelgebunden oder kabellos mit dem Beatmungsgerät verbunden. Es werden in einem gemeinsamen Trigger-Kriterium überprüft, ob die Dekompressionszeit eine Spanne von 200-340ms überschritten hat und zugleich der Atemwegsdruck einen Relativdruck von 0,9 bis 3,7mbar gegenüber einem eingestellten PEEP oder einem exspiratorischen Druckminimum überschritten hat sowie die Drucksteigung einen Wert von 25 bis 375 hPa/s übersteigt. Weiterhin wird der Trigger alternativ durch das Überschreiten eines exspiratorischen Volumens von 6 bis 20ml oder den Anstieg des exspiratorischen CO2-Wertes von 5 bis 40% über dem inspiratorischen Wert ausgelöst. Die Triggerempfindlichkeit ist in dieser Ausführung in 15 Stufen von 'empfindlich' bis 'unempfindlich' in den genannten Wertebereichen einstellbar. Die Anwendungsdauer der CCS-Beatmung mittels druckkontrollierter Beatmung mit inspiratorischen Drücken von 45 bis 60mbar beträgt zwischen 205 und 340ms und ist in 10 Stufen verstellbar. Die Voreinstellung ist für den Inspirationstrigger in der mittleren Einstellung, die Applikationsdauer beträgt in der Voreinstellung 265ms.

Der PEEP für die Anwendung beträgt in der Voreinstellung 0 mbar, die O2-Konzentration 100%. Beide Einstellungen lassen sich bedarfsgemäß nachjustieren. Das Gerät kann besonders bedienungsfreundlich mit einem einzigen Knopfdruck in den CCS-Modus geschaltet werden und bedarf keinerlei Anpassung an den Patienten. Dies ist insbesondere gegenüber konventionellen Beatmungsformen vorteilhaft, da bei diesen zunächst Einstellungen wie Tidalvolumen oder Beatmungsdruck, Frequenz, PEEP und das I:E-Verhältnis eingestellt werden müssen, um die Beatmung an den Patienten (Größe, Gewicht) und seine Kondition (Vorerkrankung, Verletzungen) anzupassen.

In einer weiteren Ausführungsform nach Fig. 6 oder Fig. 7 wird ein Gerät (15) erweitert durch ein patientenseitig angebrachtes 4/2-Wege-Ventil, welches während der Exspiration, d.h. während der Dekompression die Patientenwege mit dem Ansaugbereich des eingebauten Injektors verbindet und gleichzeitig den Gasausgang des Gerätes (15) auf die Umgebung schaltet. Während der Dekompressionsphase wird das Injektorsystem betrieben und ein entsprechender Unterdruck von 10-40mbar an den Atemwegen erzeugt, um die Dekompression optimal zu unterstützen. Zur nächsten Kompression wird das 4/2-Wege-Ventil mittels der Trigger-Algorithmen wiederum umgeschaltet und durch Zuführung von Gas die Kompression optimal unterstützt.

In einer anderen Ausführungsform nach Fig. 6 oder Fig. 7 wird ein Beatmungsgerät (15) erweitert durch eine Draht- oder Funkverbindung (22) zu einer Vorrichtung zur Thoraxkompression (30), welche die Thoraxkompressionen automatisch, halb-automatisch oder unterstützend umsetzt. Zu Beginn jeder Kompression wird ein Steuersignal an das Beatmungsgerät (15) gesendet, damit dieses eine nächste Inspiration auslöst. Dieses Steuersignal ersetzt den oben beschriebenen Trigger nur solange, wie dieses Signal zeitlich vor dem Trigger auftaucht. Somit ist der Betrieb des Beatmungsgerätes sowohl mit manueller Herzdruckmassage wie auch mit einer Vorrichtung zur Thoraxkompression (30) möglich.

Der Vorteil dieser Anwendungsform ist die optimierte Ansteuerung des Beatmungsgerätes ohne mechanische Verzögerungen, welche bei der Übertragung der Kompressionskraft auf Sternum und in der Folge auf die Druckverhältnisse in der Lunge, in den Atemwegen und an der Sensorik (23a, b) zur Auslösung des Triggers entsteht.

Eine Ergänzung zu allen Ausführungsformen sieht eine Steuerleitung (22) vor, welche Steuersignale von einem Vorrichtung zur Thoraxkompression zum Anzeigen des Beginns einer Thoraxkompression überträgt.

In einer nochmals optimierten Ausführung der Vorrichtung wird ein druckgasbetriebenes Beatmungsgerät (15), welches wahlweise mittels einer Injektoreinrichtung Gaskonzentrationen von wahlweise 60% (Airmix) oder 100% 02 ermöglicht, mittels eines Einschlauchsystems (5) mit patientennahem Exspirationsventil an einen Patienten konnektiert. Das Beatmungsgerät kann mit Hilfe seiner patientennah angebrachten Drucksensorik (23) den Beginn einer Thoraxkompression messen, indem relative Druckerhöhungen und positive Drucksteigungen erfasst werden. Dazu werden in einem gemeinsamen Trigger-Kriterium überprüft, ob die Dekompressionszeit eine Spanne von 270ms überschritten hat und zugleich der Atemwegsdruck einen Relativdruck von 2,3mbar gegenüber einem exspiratorischen Druckminimum überschritten hat sowie die Drucksteigung einen Wert von 200hPa/s übersteigt. Die Anwendungsdauer der CCS-Beatmung mittels volumenkontrollierter Beatmung beträgt zwischen 265ms.

Das Gerät kann besonders bedienungsfreundlich mit einem einzigen Knopfdruck in den CCS-Modus (24) geschaltet werden und bedarf lediglich der Anpassung des inspiratorischen Flusses an den Patienten durch Verstellung des Minutenvolumens oder Tidalvolumens. Eine Einstellung erfolgt derart, dass während der Reanimation der angezeigte Atemwegsdruck 60mbar nicht überschreitet.

In einer weiteren Auführungsform wird als Gasquelle ein Gebläse (15) und/oder eine druckgasbetriebene Sauerstoffquelle (15) genutzt, bevorzugt angesteuert mittels eines Proportionalventils. Das Gerät vermag O2-Konzentrationen im Bereich 21-100% zu verabreichen und ist über ein Zwei- bzw. Doppelschlauchsystem (5) mit dem Patienten verbunden. Das Beatmungsgerät kann mit Hilfe seiner patientennah angebrachten Flow-, und/oder Volumen-, und/oder CO2- und/oder Drucksensorik (23b) den Beginn einer Thoraxkompression messen, indem relative Druckerhöhungen, positive Drucksteigungen, exspiratorische Flüsse oder exspiratorische Volumina oder CO2-Konzentrationen erfasst werden. Dazu werden in einem gemeinsamen Trigger-Kriterium überprüft, ob die Dekompressionszeit eine Spanne von 200-340ms überschritten hat und zugleich der Atemwegsdruck einen Relativdruck von 0,9 bis 3,7mbar gegenüber einem eingestellten PEEP oder einem exspiratorischen Druckminimum überschritten hat sowie die Drucksteigung einen Wert von 25 bis 375hPa/s übersteigt. Weiterhin wird der Trigger alternativ durch das Überschreiten eines exspiratorischen Volumens von 6 bis 20ml oder den Anstieg des exspiratorischen CO2-Wertes von 5 bis 40% über dem inspiratorischen Wert ausgelöst. Die Triggerempfindlichkeit ist in dieser Ausführung in 15 Stufen von 'empfindlich' bis 'unempfindlich' in den genannten Wertebereichen einstellbar.

Die Anwendungsdauer der CCS-Beatmung mittels druckkontrollierter Beatmung mit inspiratorischen Drücken von 45 bis 60mbar beträgt zwischen 205 und 340ms und ist in 10 Stufen verstellbar. Die Voreinstellung ist für den Inspirationstrigger in der mittleren Einstellung, die Applikationsdauer beträgt in der Voreinstellung 265ms.
Der PEEP für die Anwendung beträgt in der Voreinstellung 0 mbar, die O2-Konzentration 100%. Beide Einstellungen lassen sich bedarfsgemäß nachjustieren.

Das Gerät kann besonders bedienungsfreundlich mit einem einzigen Knopfdruck in den CCS-Modus (24) geschaltet werden und bedarf keinerlei Anpassung an den Patienten. Dies ist insbesondere gegenüber konventionellen Beatmungsformen vorteilhaft, da bei diesen zunächst. Einstellungen wie Tidalvolumen oder Beatmungsdruck, Frequenz, PEEP und das I:E-Verhältnis eingestellt werden müssen, um die Beatmung an den Patienten (Größe, Gewicht) und seine Kondition (Vorerkrankung, Verletzungen) anzupassen. Weiterhin ist die Aktivierung der CCS-Beatmung in einen Benutzerdialog eingebunden, der auf die Behandlung von reanimationspflichtigen Patienten abgestimmt ist in dem Sinne, dass der Prozess der Reanimation optimal unterstützt wird und gleichzeitig möglichst wenig Ablenkung durch das Gerät erfolgt. Dazu wird das Gerät eingeschaltet und zunächst in einem Modus zur Maskenbeatmung betrieben. Dieser Modus entspricht dem CCS-Modus wie oben dargestellt mit einem verminderten Beatmungsdruck von 20mbar. Durch den Anwender lässt sich diese Betriebsart auf eine konventionelle Beatmung mittels Maske nach den Algorithmen der Leitlinien umstellen: z.B. 30 Herzdruckmassagen gefolgt von 2 Beatmungshüben (aus den Richtlinien der ERC von 2010). Als zweite Phase für die optimale Unterstützung der Beatmung wird durch einen einzigen Knopfdruck (25) eine Präoxygenierungsphase als Vorbereitung für die Intubation umgesetzt. Hierzu wird entweder ein CPAP-Modus aktiviert oder eine flowkonstante 02-Abgabe durchgeführt. Die Wahl des Verfahrens kann durch den Anwender einstellt werden. Als dritte Phase wird zur Durchführung der Intubation der Gasfluss vom Gerät durch einen Knopfdruck (26) deaktiviert. Schließlich wird nach erfolgter Intubation durch einen Knopfdruck (24) auf den CCS-Modus umgeschaltet, so dass hier eine optimale Unterstützung der Thoraxkompressionen mit 60mbar Inspirationsdruck erfolgt. Der CCS-Modus beginnt mit einem bis drei Testhüben, um die Thorax-Eigenschaften mittels der patientennah angebrachten Sensoren zu erfassen. Dann wird der CCS-Modus erfindungsgemäß durch die Herzdruckmassage ausgelöst.

Während der gesamten Wiederbelebung wird erfindungsgemäß permanent über die Druck-, Flow-, Volumen- und CO2-Schwankungen sensorisch (23b) die Frequenz der Herzdruckmassage gemessen und parallel zum Benutzerdialog angezeigt (21). Bei Abweichungen vom Sollwert (jeweils aktualisiert nach den Richtlinien) werden Hinweise auf der Anzeige (21) des Beatmungsgerätes wie auch hörbar (29) ausgegeben. Dazu kann vom Anwender des Gerätes eingestellt werden, ob die akustischen Rückmeldungen in Form von Sprachausgaben oder höhenveränderlichen Tönen erfolgt. Erfindungsgemäß wird eine Vorrichtung zur Unterstützung eines optimierten Ablaufs während der Reanimation vorgeschlagen, bei der der Anwender das Gerät lediglich einschalten muss und dann durch einfache Knopfdrücke von einer Phase der Reanimation zur nächsten geleitet wird. Hierbei wird unterschieden zwischen einer ersten Phase der Reanimation mit Maskenbeatmung; die hierzu notwendige Beatmung wird mit Einschalten des Beatmungsgerätes aktiviert. Eine wahlweise zweite Phase zur Vorbereitung der Intubation des Patienten wird durch einen einfachen Knopfdruck aktiviert, so dass der Patient präoxygeniert wird. In einer wahlweisen dritten Phase wird die Gaszufuhr vom Beatmungsgerät durch einen einfachen Knopfdruck deaktiviert, damit störungsfrei und ohne 02-Verbrauch intubiert werden kann. In einer letzten Phase wird durch die Vorrichtung das Verfahren zur Unterstützung der Thoraxkompression aktiviert.

Die Umsetzung der Benutzerführung erfolgt mittels einer graphischen Bedienoberfläche, welche durch eindeutige Texte und/oder graphische Symbole die Bedienelemente zum Aktivieren der jeweils nächsten Phase kennzeichnen. In jeder Phase des geführten Ablaufs besteht die Option zur jeweils vorherigen Phase des Ablaufs zurück zu kehren. Dazu sind eindeutige Kennzeichnungen des Bedienelementes zum Auslösen dieser Rückkehr vorgesehen. Die Aktivierung der jeweils nächsten Phase wird bevorzugt durch einen Sicherheitsmechanismus bestätigt. Der Sicherheitsmechanismus wird durch das zweimalige Drücken des Aktivierungsknopfes innerhalb einer Zeit von 3 Sekunden umgesetzt. Alternativ wird der Sicherheitsmechanismus umgesetzt durch das zusätzliche Drücken eines weiteren dazu bestimmten und gekennzeichneten Bedienelementes.

## Patentansprüche

1. Vorrichtung zur Verwendung während der Herz-Druckmassage (HDM) eines Patienten, wobei die ·Vorrichtung aufweist: eine Atemgasquelle (15) und Mittel um Atemgas zum Atemweg des Patienten zu leiten (5, 9), sowie Sensormittel (23a, 23b) um eine Thoraxkompression festzustellen, und die zudem Steuermittel aufweist, welche einen Atemgashub auslösen, wenn die Sensormittel (23a, 23b) eine Thoraxkompression registriert haben, **dadurch gekennzeichnet, dass** die Atemgasquelle (15) ausgebildet ist, den Atemgashub während einer Thoraxkompression im wesentlichen aufrecht zu erhalten, und die Vorrichtung zur Vorgabe eines PEEP-Druckes in der Zeitspanne bis zur nächsten Kompression ausgebildet ist, um eine kontrollierte Entlastung der Atemwege zu gewährleisten.

2. Vorrichtung nach Anspruch 1. **dadurch gekennzeichnet, dass** das Steuermittel ausgebildet ist, den Atemgashub zeitversetzt oder zeitgesteuert auszulösen.

3. Vorrichtung nach Anspruch 1. **dadurch gekennzeichnet, dass** das Sensormittel (23a) als Sensor ausgeführt ist, welcher ausgebildet ist, mechanische Drucksignale im Bereich des Thorax eines Patienten zu registrieren und über eine Steuerleitung (22) an die Atemgasquelle (15) zu leiten.

4. Vorrichtung nach Anspruch 1. **dadurch gekennzeichnet, dass** das Sensormittel (23b) als Sensor ausgeführt ist, welcher Atemgasparameter registriert.

5. Vorrichtung nach einem der vorhergenenden Ansprüche **dadurch gekennzeichnet, dass** das Sensormittel (23a, 23b) ausgebildet ist, erst bei Überschreiten einer Triggerschwelle, Signale weiterzuleiten.

6. Vorrichtung nach einem der vorhergenenden Ansprüche **dadurch gekennzeichnet, dass** ein Atemgashub gerätegesteuert ausgelöst wird, wenn die Vorrichtung zur Thoraxkompression (30) ein Steuersignal an die Atemgasquelle (15) leitet.

7. Vorrichtung nach einem der vorhergenenden Ansprüche **dadurch gekennzeichnet, dass** im Bereich der Vorrichtung eine Signaleinrichtung (29) vorgesehen ist, welche die Frequenz für die HDM vorgibt oder die Frequenz einer HDM wiedergibt.

8. Vorrichtung nach einem der vorhergenenden Ansprüche **dadurch gekennzeichnet, dass** die Thoraxkompression durch einen Atemgashub bedingt ist.

9. Vorrichtung nach einem der vorhergenenden Ansprüche **dadurch gekennzeichnet, dass** ein Atemgashub erst ausgelöst wird, nachdem Sensormittel (23a, 23b) die Beendigung einer Thoraxkompression registriert haben.

10. Vorrichtung nach einem der vorhergenenden Ansprüche **dadurch gekennzeichnet, dass** das Steuermittel ausgebildet ist, einen Steuerimpuls für die Signaleinrichtung (29) zu generieren.

11. Vorrichtung nach einem der vorhergenenden Ansprüche **dadurch gekennzeichnet, dass** das Steuermittel ausgebildet ist, einen Steuerimpuls für die Vorrichtung zur Thoraxkompression (30) zu generieren.

12. Vorrichtung nach einem der vorhergenenden Ansprüche mit einem Sensormittel (23a, 23b) zur Verwendung während der Herz-Druckmassage (HDM) eines Patienten **dadurch gekennzeichnet, dass** das Sensormittel eine Thoraxkompression registriert und erst bei Überschreiten einer Triggerschwelle Signale über eine Steuerleitung (22) weiterleitet.

## Claims

1. A device for use during cardiac massage (CM) of a patient, wherein the device has: a breathing gas source (15) and means for conducting breathing gas to the airway of the patient (5, 9), as well as sensor means (23a, 23b) for detecting a chest compression, and which moreover has control means which trigger a breathing gas stroke when the sensor means (23a, 23b) have registered a chest compression, **characterised in that** the breathing gas source (15) is designed to substantially maintain the breathing gas stroke during a chest compression and the device is designed to specify a PEEP pressure in the time interval until the next compression in order to ensure a controlled pressure-relief of the airways.

2. A device according to Claim 1, **characterised in that** the control means is designed to trigger the breathing gas stroke in time-delayed or time-controlled manner.

3. A device according to Claim 1, **characterised in that** the sensor means (23a) is constructed as a sensor which is designed to register mechanical pressure signals in the chest region of a patient and to conduct them to the breathing gas source (15) by way of a control line (22).

4. A device according to Claim 1, **characterised in that** the sensor means (23b) is constructed as a sensor which registers breathing gas parameters.

5. A device according to one of the preceding claims, **characterised in that** the sensor means (23a, 23b) is designed to transmit signals only when a trigger threshold is exceeded.

6. A device according to one of the preceding claims, **characterised in that** a breathing gas stroke is triggered in equipment-controlled manner when the chest-compression device (30) conducts a control signal to the breathing gas source (15).

7. A device according to one of the preceding claims, **characterised in that** a signal mechanism (29), which reproduces the frequency for the CM or the frequency of a CM, is provided in the region of the device.

8. A device according to one of the preceding claims, **characterised in that** the chest compression is contingent on a breathing gas stroke.

9. A device according to one of the preceding claims, **characterised in that** a breathing gas stroke is only triggered after sensor means (23a, 23b) have registered the end of a chest compression.

10. A device according to one of the preceding claims, **characterised in that** the control means is designed to generate a control pulse for the signal mechanism (29).

11. A device according to one of the preceding claims, **characterised in that** the control means is designed to generate a control pulse for the chest-compression device (30).

12. A device according to one of the preceding claims, having a sensor means (23a, 23b) for use during the cardiac massage (CM) of a patient, **characterised in that** the sensor means registers a chest compression and transmits signals by way of a control line only when a trigger threshold is exceeded.

## Revendications

1. Dispositif destiné à être utilisé pendant le massage cardiaque par compression (HDM) d'un patient, sachant que le dispositif est doté d'une source de gaz respiratoire (15) et de moyens (5, 9) pour conduire le gaz respiratoire aux voies respiratoires du patient, ainsi que de moyens de détection (23a, 23b) pour le captage d'une compression thoracique, et qui est pourvu, de plus, de moyens de commande, qui déclenchent une pulsation de gaz respiratoire quand les moyens de détection (23a, 23b) ont enregistré une compression thoracique, **caractérisé en ce que** la source de gaz respiratoire (15) est conçue pour maintenir sensiblement la pulsation de gaz respiratoire pendant une compression thoracique et q u e le dispositif de prédétermination d'une pression PEEP est conçu pour assurer un soulagement contrôlé des voies respiratoires.

2. Dispositif selon la revendication 1, **caractérisé en ce que** le moyen de commande est conçu pour déclencher la pulsation de gaz respiratoire en temps décalé ou en temps commandé.

3. Dispositif selon la revendication 1, **caractérisé en ce que** le moyen de détection (23a) est réalisé sous la forme d'un capteur qui est conçu pour enregistrer des signaux de compression mécaniques dans la région du thorax d'un patient et pour les transmettre à la source de gaz respiratoire (15) par l'intermédiaire d'une conduite de commande (22).

4. Dispositif selon la revendication 1, **caractérisé en ce que** le moyen de détection (23b) est réalisé sous la forme d'un capteur qui est conçu pour enregistrer des paramètres de gaz respiratoire.

5. Dispositif selon l'une des revendications précédentes,
**caractérisé en ce que** les moyens de détection (23a, 23b) sont conçus pour ne transmettre des signaux que lors du dépassement d'un seuil de déclenchement.

6. Dispositif selon l'une des revendications précédentes
**caractérisé en ce qu**'**une** pulsation de gaz respiratoire est déclenchée par le système de commande quand le dispositif lance à la source de gaz respiratoire (15) un signal de commande de compression thoracique.

7. Dispositif selon l'une des revendications précédentes,
**caractérisé en ce que**, dans la région du dispositif, est prévue une installation de signalisation (29), qui prédétermine la fréquence pour le massage cardiaque par compression (HDM) ou représente la fréquence d'un massage cardiaque par compression (HDM).

8. Dispositif selon l'une des revendications précédentes
**caractérisé en ce que** la compression thoracique est déterminée par une pulsation de gaz respiratoire.

9. Dispositif selon l'une des revendications précédentes
**caractérisé en ce qu I** une pulsation de gaz respiratoire n'est déclenchée que des moyens de détection (23a, 23b) ont enregistré la fin d'une compression thoracique.

10. Dispositif selon l'une des revendications précédentes
**caractérisé en ce que** le moyen de détection est conçu pour générer une impulsion de commande pour le système de signalisation (29).

11. Dispositif selon l'une des revendications précédentes,
**caractérisé en ce que** le moyen de détection est conçu pour générer une impulsion de commande pour le dispositif de compression thoracique (30).

12. Dispositif selon l'une des revendications précédentes qui est doté d'un moyen de détection (23a, 23b) Destiné à 1etre utilisé pendant le massage cardiaque par compression (HDM) d'un patient,
**caractérisé en ce que** le moyen de détection enregistre une compression thoracique et ne transmet des signaux, par l'intermédiaire d'une conduite de commande (22), que lorsqu'un seuil de déclenchement est dépassé.
